# EUROPEAN PATENT SPECIFICATION

(11) **EP 4 340 915 B1**
(45) Date of publication and mention of the grant of the patent: **05.03.2025**
(21) Application number: 22729185.3
(22) Date of filing: 16.05.2022
(51) Int. Cl.: A61M 15/00, G16H 20/13

(54) **ADHERENCE MONITOR FOR A DRY POWDER INHALER**
EINHALTUNGSMONITOR FÜR EINEN TROCKENPULVERINHALATOR
MONITEUR D'ADHÉRENCE POUR UN INHALATEUR DE POUDRE SEC

(30) Priority: 17.05.2021 EP 21174144
(43) Date of publication of application: 27.03.2024
(73) Proprietor: Vectura Delivery Devices Limited, Chippenham, Wiltshire SN14 6FH (GB)
(72) Inventor: COTTON, Darryl, Cambridge Cambridgeshire CB4 0GZ (GB)
(74) Representative: Clarke, Christopher John
(86) International application number: PCT/EP2022/063133
(87) International publication number: WO 2022/243219

(56) References cited:
- WO-A1-2015/178907
- WO-A2-2014/006135
- GB-A- 2 502 350

## Description

### Technical Field of the Invention

The present invention relates to an adherence monitor for a blister strip dry powder inhaler. In particular, it relates to an adherence monitor with an optical sensor with a light guide for reading a non-numerical code on the blister strip.

### Background to the Invention

Dry powder inhalers (DPIs) provide an attractive method for administering medicaments, for example to treat local diseases of the airway or to deliver drugs to the bloodstream via the lungs. The medicament is commonly provided as individual doses, such as a strip having a plurality of blisters, for example as disclosed in WO13/175177. The efficacy of treatment is dependent on the patient using the inhaler as prescribed. Consequently, there is increasing interest in monitoring patient adherence, i.e. whether the patient takes the prescribed number of doses per day, e.g. once or twice daily.

DPIs typically have a dose counter, either in the form of numbers printed onto the blister strip or as a separate mechanism which counts up or down each time the inhaler is actuated. However, while this tells the patient the number of remaining doses so that they know when a new inhaler is required, it does not help the caregiver to monitor adherence, because the dose number is only seen by the patient. Therefore, monitors have been developed that provide adherence information. The monitor typically has switches or sensors (e.g. optical, or acoustic) which detect when the inhaler has been actuated, and hence counts the number of doses that have been taken. However, if an actuation is not counted correctly (for example, an aborted actuation is counted, or an actuation is not counted due to sensor error) then monitor could display the incorrect dose number.

Since adherence monitors typically contain expensive sensors, electronics etc., they are often provided as separate add-on modules which couple to the inhaler. DPIs typically contain a month's supply of medication. Thus, when the medication in the inhaler has been used up, the monitor can be detached and then re-attached to a new inhaler. For example, US2010/0192948 discloses a detachable adherence monitor for a DPI which records when the inhaler is used by optically detecting movement of its mechanical parts. Since the monitor is detachable, the patient could remove it before all of the doses in the inhaler are used up. The monitor has the disadvantage that if it is later re-attached to the same inhaler, it cannot determine how many doses have been dispensed during the period in which it was detached. WO2015/178907 discloses a usage monitoring device that can be selectively coupled to one of a variety of different inhalers.

Our co-pending application under Art 54(3) EPC, EP4061454 (derived from WO2021/099329) seeks to overcome these drawbacks by providing a dry powder inhaler which contains a blister pack (such as a blister strip) comprising a plurality of blisters. The blister pack has non-numerical indicia which encode a number that is associated with each dose of powdered medicament. The dose number is unique within the blister strip. The inhaler is adapted for mounting a monitor, which has optical sensor(s) that read the non-numerical indicia on the blister pack. The monitor interprets the output from the optical sensor(s) from which it determines the dose number. Thus there is no possibility of the dose number being incorrect. In other words, the monitor determines the dose number in an absolute manner by reading the dose number from the blister strip, rather than in a relative manner, such as by counting uses of the inhaler as in US2010/0192948.

### Brief description of the invention

The present invention relates to the design of the optical system in this type of monitor. In a first aspect, the invention provides a monitor which is attachable to a dry powder inhaler that contains a blister strip comprising a plurality of blisters, wherein a blister, or group of blisters, provides a dose of powdered medicament for inhalation, wherein the blister strip has a plurality of non-numerical indicia; wherein the monitor comprises one or more optical sensors which are configured to read the non-numerical indicia; wherein the optical sensor has a light guide with a proximal end adjacent to the sensor and a distal end remote from the sensor through which light is transmitted to and received from the blister strip, wherein the distal end is convex.

The term "light guide" means a device for directing light by means of total internal reflection from a light source to a place where the light is needed. Thus a light guide differs from a transparent window which transmits light, but does not guide or direct it by total internal reflection. The proximal end of the light guide(s) which is adjacent to the optical sensor may be wider than the distal end, and the sides of the light guide(s) may be sloped, so that the light guide may have a generally trapezoidal (in particular, isosceles trapezoidal) cross-section, apart from the convex distal end. The ratio of the width of the proximal end of the light guide(s) to the width of the distal end of the light guide may be from 4:3 to 3:1, such as from 3:2 to 5:2, preferably about 2:1.

The width of the proximal end may be from 2 to 3 mm, such as about 2.4 mm; and the width of the distal end may be from 1 to 1.5 mm, such as about 1.2 mm.

The convex distal end of the light guide may have a radius of curvature of from 2 to 5 mm, such as about 3mm.

The light guide(s) may have a height of from 2 to 3 mm, such as about 2.5 mm. The light guide(s) may have a depth of from 1 to 3 mm, such as about 1.7 mm.

The monitor may be removably attachable to the inhaler.

The optical sensor(s) may be photomicrosensors which emit and detect light reflected from the indicia on the blister strip. The optical sensor(s) may comprise a single light source, such as an LED, and two or more photodetectors.

The optical sensor(s) may operate in the visible and / or infrared regions of the electromagnetic spectrum.

The light guide(s) may be made from a transparent plastic material, such as acrylic (also known as polymethyl methacrylate or plexiglass) or polycarbonate. The light guide(s) may be built in to the housing of the monitor (i.e. they are moulded together), in which case the light guide is preferably made from polycarbonate.

In one embodiment, the monitor has three photomicrosensors and three light guides. The light guides may be moulded as a single component.

In a second aspect, the present invention provides a kit comprising a monitor of the first aspect and a dry powder inhaler which contains a blister strip comprising a plurality of blisters, wherein the blister strip has non-numerical indicia, and wherein the inhaler has an aperture into which the light guide(s) are inserted when the monitor is mounted onto the inhaler.

In a third aspect, the present invention provides a combination comprising a monitor according to the first aspect which is mounted on a dry powder inhaler that contains a blister strip comprising a plurality of blisters, wherein the blister strip has non-numerical indicia, and wherein the light guide(s) on the monitor are inserted into an aperture on the inhaler, so that the optical sensors can read the non-numerical indicia.

The monitor may be removably mounted on the inhaler, for example it may clip onto the housing of the inhaler so that a single monitor may be used with many different inhalers. Alternatively, the monitor may be intended for use with a single inhaler only, in which case it may be permanently attached to the inhaler, e.g. by ultrasonic welding or gluing.

The non-numerical indicia may each encode an individual, unique number that is associated with a dose of powdered medicament provided by a blister, or a group of blisters. The monitor reads the indicium on the blister strip associated with the current dose. From this it determines the unique number of that dose, and consequently the number of doses that have been dispensed or remain to be dispensed.

### Brief Description of the Figures

The invention will now be further described with reference to the Figures, wherein:
Figure 1A shows an inhaler and a monitor according to the invention, with the cover in the closed position, so that the mouthpiece is covered.
Figure 1B shows the inhaler with the cover in the open position.
Figure 1C shows another view of the inhaler from a different angle.
Figure 1D shows the inhaler with the monitor removed and the cover closed.
Figure 1E is a cross-section through the inhaler and monitor.
Figures 2A and 2B show the monitor removed from the inhaler.
Figure 3 shows a blister strip with indicia in the form of 2D matrix codes.
Figure 4 shows a 2D matrix code.
Figure 5 shows a an optical sensor with a light guide inserted into the aperture of the inhaler.
Figure 6 shows a perspective view of a light guide.
Figure 7 shows voltage outputs from the optical sensors for four different codes.

### Detailed Description of the Invention

In the context of dry powder inhalers, the term "adherence" is normally used to refer to whether the patient takes the prescribed number of doses per day, e.g. once or twice daily. The term "compliance" is normally used to refer to whether the patient uses their inhaler correctly, e.g. if they inhale sufficiently strongly to entrain the powder and disperse it into particles that reach the lung. Various types of sensor can be used to measure adherence and / or compliance information. In the present application, the term "monitor" refers to a module having one or more sensors that is designed to measure and capture information relating to adherence, and which may additionally measure and capture compliance information. The monitor does not perform any of the functions associated with dosing the medication, such as a piercing or opening blisters, de-agglomerating the powder or providing a breath-actuation mechanism. The inhaler therefore operates to dispense powder whether the monitor is present or not.

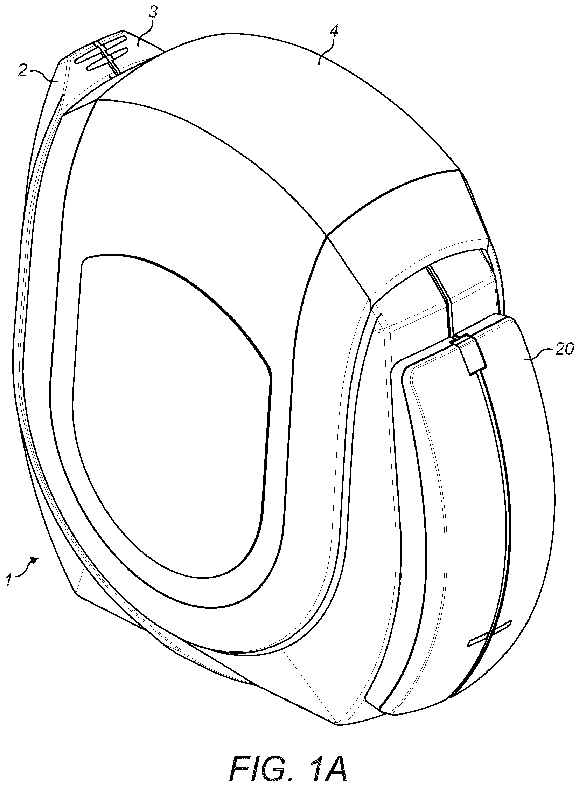

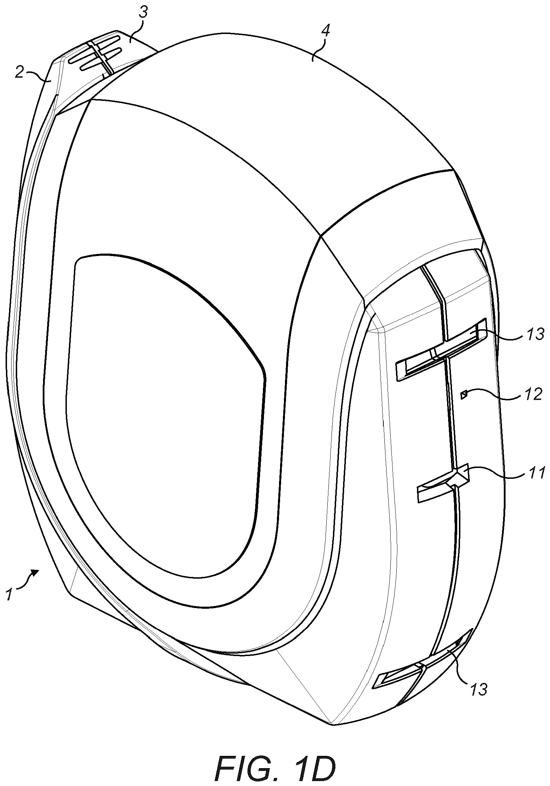

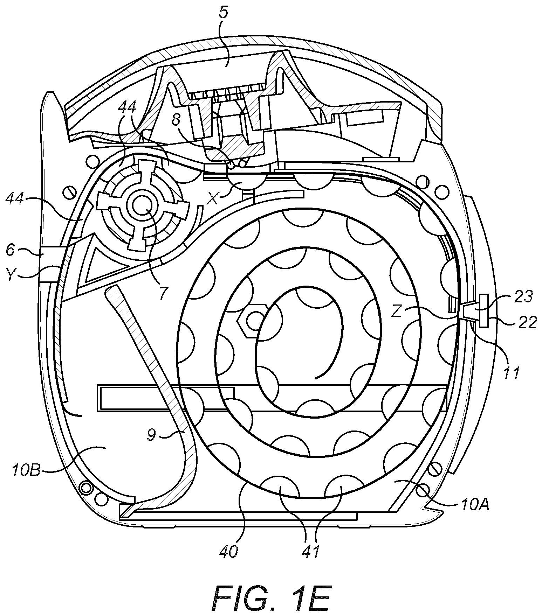

Figures 1A-1C and 1E show an inhaler with a monitor according to the invention. Figure 1D shows the inhaler without the monitor. The inhaler is an "open-inhale-close" inhaler of the type described in WO13/175177. However, the invention is not limited to this type of inhaler, and, for example, could equally be used with an inhaler which has a passive mouthpiece cover and a separate actuating lever, as described for example in WO13/175176.

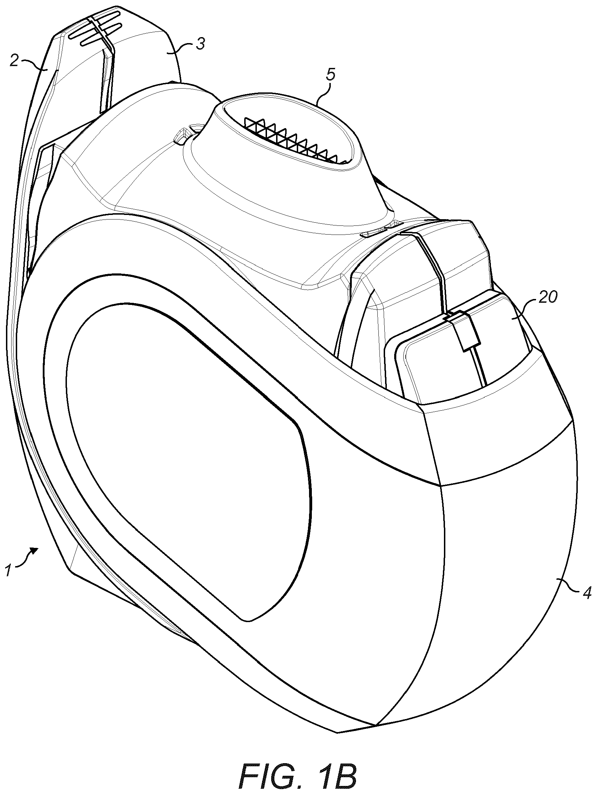

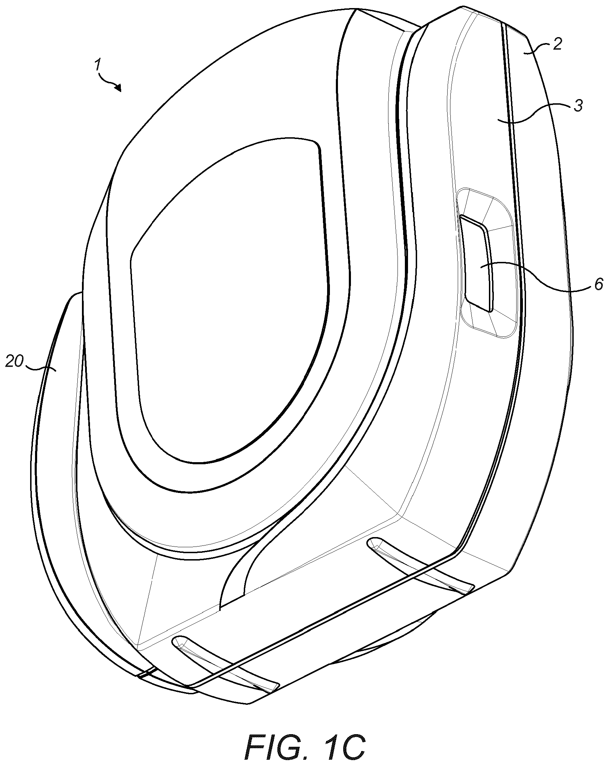

The inhaler **1** is constructed from two shell parts **2,** 3 which are joined together to form a housing that contains a blister strip. A detachable monitor **20** is attached to one side of the inhaler. A mouthpiece cover **4** is mounted onto the housing. The cover **4** can be rotated through an angle of about 100° from the closed position (Figure 1A) in which it covers and protects a mouthpiece **5** to a fully open position (Figure 1B), in which the mouthpiece is exposed so that the user can inhale a dose of medicament. As can been seen in Figure 1C, the housing has an opening **6** on the opposite side from the monitor **20.** This allows the user to see a number printed on the blister strip. When the user actuates the inhaler by opening the cover, the indexing mechanism advances the blister strip by one dose. As a result, the number on the blister strip that is visible to the user sequentially decreases (or increases). The numbers therefore provide a dose counter which displays the number of doses remaining (or that have been dispensed) in the inhaler.

Figure 1D shows the inhaler with the monitor having been removed and with the cover closed. An aperture **11** is visible in the wall of the housing where the monitor was attached. The aperture allows the monitor to read the indicia on the blister strip. The aperture is spaced apart from the dispensing position, in the 'upstream' direction (i.e. before the blister is opened), so that the indicia that are read by the monitor are on the unused part of the blister strip. However, the aperture could be located at or near the dispensing position, or 'downstream' (i.e. after the blister has been opened) of the dispensing position, provided that the blister strip is arranged to take this into account, as is explained below. The latter is less preferred, since at least in theory, there could be traces of powder on the used part of the blister strip, in particular on the indicia, which could lead to errors in reading them.

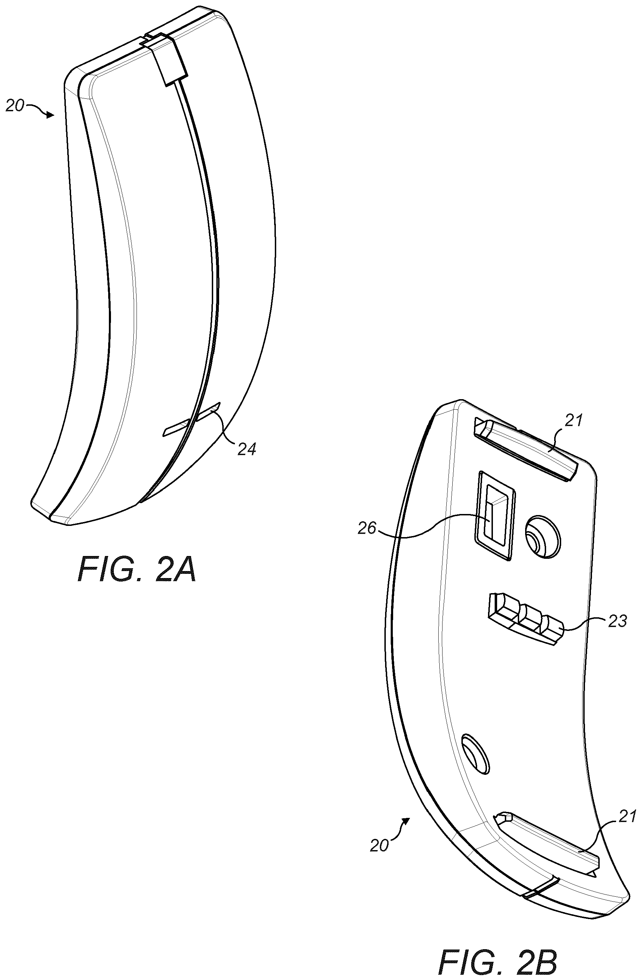

There is also a small orifice **12** in the wall of the housing, which allows a pressure sensor **26** (shown in Figure 2B) in the monitor to connect to the mouthpiece via a channel inside the inhaler. The housing also has two slots **13** for mounting the monitor as described below.

Figure 1E is a cross-section through the inhaler and the monitor. The inhaler contains a strip **40** of blisters **41** containing powdered medicament for inhalation. The blister strip is typically cold-formed from a ductile foil laminate or a plastics material and has a pierceable lid, typically foil (e.g. aluminium) or a foil laminate, which is heat-sealed around the periphery of the blisters after the dose of medicament has been introduced during manufacture.

The inhaler has a mechanism for indexing the blister strip and a piercer for opening the blisters. The mouthpiece **5** is formed as part of a component which is pivotally mounted to the housing and the piercer **8** is mounted on the underside of the mouthpiece. The cover (not shown in Figure 1E) is selectively coupled to the indexing mechanism and to the mouthpiece component. Moving the cover from the closed position to an intermediate position (the first stage of opening) causes the indexing mechanism to advance the blister strip. Then, once an unused blister **X** is in position beneath the piercer **8,** the indexing mechanism is disengaged. The first stage is reversible, i.e. the user can abort the actuation of the inhaler (since piercing has not yet occurred) simply by closing the cover, which moves the blister strip back to its previous position. Moving the cover from the intermediate position to the fully open position (the second stage) causes the mouthpiece component to pivot downwards so that the piercer pierces the aligned blister. The user then inhales through the mouthpiece, which aerosolizes the powder in the pierced blister.

The inhaler may be configured to index and pierce one blister on each actuation. Alternatively, it may index and pierce two (or more) blisters on each actuation, and thereby deliver two (or more) different formulations simultaneously, or a double (or multiple) amount of a single formulation. Thus a dose of medicament may be provided by a single blister, in which case an individual non-numerical indicium is associated with each blister. Alternatively, a dose may be provided by two (or more) blisters, in which case an individual non-numerical indicium is associated with each pair (or group) of blisters.

The medicament is suitable for administration by inhalation, for example for the treatment of a respiratory disease. It may include one of more of the following classes of pharmaceutically active material: anticholinergics, adenosine A2A receptor agonists, β2-agonists, calcium blockers, IL-13 inhibitors, phosphodiesterase-4-inhibitors, kinase inhibitors, steroids, CXCR2, proteins, peptides, immunoglobulins such as Anti-IG-E, nucleic acids in particular DNA and RNA, monoclonal antibodies, small molecule inhibitors and leukotriene B4 antagonists. The medicament may include excipients, such as fine particles and / or carrier particles (e.g. lactose), and / or additives (e.g. magnesium stearate, phospholipid or leucine).

Suitable β2-agonists include albuterol (salbutamol), preferably albuterol sulfate; carmoterol, preferably carmoterol hydrochloride; fenoterol; formoterol; milveterol, preferably milveterol hydrochloride; metaproterenol, preferably metaproterenol sulfate; olodaterol; procaterol; salmeterol, preferably salmeterol xinafoate; terbutaline, preferably terbutaline sulphate; vilanterol, preferably vilanterol trifenatate and indacaterol, preferably indacaterol maleate. Suitable steroids include budesonide; beclamethasone, preferably beclomethasone dipropionate; ciclesonide; fluticasone, preferably fluticasone furoate; mometasone, preferably mometasone furoate. Suitable anticholinergics include: aclidinium, preferably aclidinium bromide; glycopyrronium, preferably glycopyrronium bromide; ipratropium, preferably ipratropium bromide; oxitropium, preferably oxitropium bromide; tiotropium, preferably tiotropium bromide; umeclidinium, preferably umeclidinium bromide; Darotropium bromide; and tarafenacin.

The active material may include double or triple combinations such as salmeterol xinafoate and fluticasone propionate; budesonide and formoterol fumarate dehydrate,lycopyrrolate and indacaterol maleate; glycopyrrolate, indacaterol maleate and mometasone furoate; fluticasone furoate and vilanterol; vilanterol and umeclidinium bromide; fluticasone furoate, vilanterol and umeclidinium bromide.

Figure 2 shows the monitor **20** on its own (i.e. detached from the inhaler). Figure 2A shows the outer side of the monitor, and Figure 2B shows the inside face of the monitor (i.e. the side which abuts the inhaler when the monitor is attached). The monitor **20** has two clips **21** which fit into the corresponding slots **13** in the housing, and thereby hold the monitor in place when attached to the inhaler. The clips and slots allow the monitor to be detachably mounted on the inhaler, e.g. by an interference fit. A detachable monitor has the advantage that it may be supplied separately from the inhaler, and that a single monitor may be used with many different inhalers. Thus, when the medication in the inhaler has been used up, the monitor can be detached and then re-attached to a new inhaler. The new inhaler could be identical to the used one. Alternatively, it could contain a different dose strength or a different active, or even be a different type of inhaler, provided that it is compatible with the monitor.

The monitor may have a pressure sensor **26,** which is located in a recess on the inside face. The pressure sensor abuts the orifice **12** in the housing (see Figure 1D), which leads via a channel in the inhaler housing to the mouthpiece. Alternatively, the pressure sensor could be connected to the mouthpiece via a separate external tube. The monitor can thereby measure the pressure in the mouthpiece to sense the user's inhalation.

The monitor has a power source, such as a rechargeable battery. The monitor may have a motion sensor, such as an accelerometer, and means for switching on the monitor when motion is detected. The motion sensor may be configured to sense a specific gesture, such as picking up the monitor. Alternatively, a reed switch and a corresponding magnet on the mouthpiece cover, or a mechanical switch which interacts with the cover can be used to switch the monitor on. This avoids the need for the monitor to be permanently switched on, and hence conserves battery power.

The monitor has a controller and memory (e.g. a suitable microprocessor) which are configured to process and/or store information from the sensors and / or switches relating to patient's usage of the inhaler. The monitor may also include means for transmitting information from the sensors and / or switches to an external device, such as a computer or smartphone, e.g. via Bluetooth^{®}. The information may then be displayed to the user and / or a medical professional, by means of suitable software, for example a smartphone app. The information may additionally or alternatively be stored on the monitor for subsequent interrogation, and / or transmitted to an online health platform. The monitor may also include means for receiving information from an external device.

The monitor has one or more optical sensors for reading the indicia on the blister strip by means of reflected light. It can then determine the unique identity of the particular blister in a manner that is described in detail below, so that the number of doses that have been dispensed or that remain to be dispensed can be determined directly from the blister strip. In the embodiment shown in Figure 2, the monitor has three optical sensors which are described in further detail below. Each sensor has a light guide **23** which protrudes beyond the face of the of the monitor. When the monitor is attached to the inhaler, the light guides fit into the aperture **11** in the inhaler housing. This has the advantage that the light guides help to locate the monitor as it is mounted on the inhaler, so that the sensors are correctly positioned with respect to the blister strip.

The sensors are separated from the blister strip by the thickness of the housings of the monitor and the inhaler, which may typically be a distance of about 2 mm. The aperture in the housing of the inhaler must be narrow in order to ensure that external objects cannot be inadvertently inserted by the user if the monitor is not present. The optical sensor emits light over a range of angles. Without a light guide, a substantial amount of the light would not enter the aperture. Instead it would be reflected back to the sensor from the housing of the inhaler. This would increase the background light and reduce the ratio of the signal (i.e. the light reflected from the blister strip) to noise (i.e. the background). The light guide counteracts this by channeling light from the optical sensor to the blister strip and back.

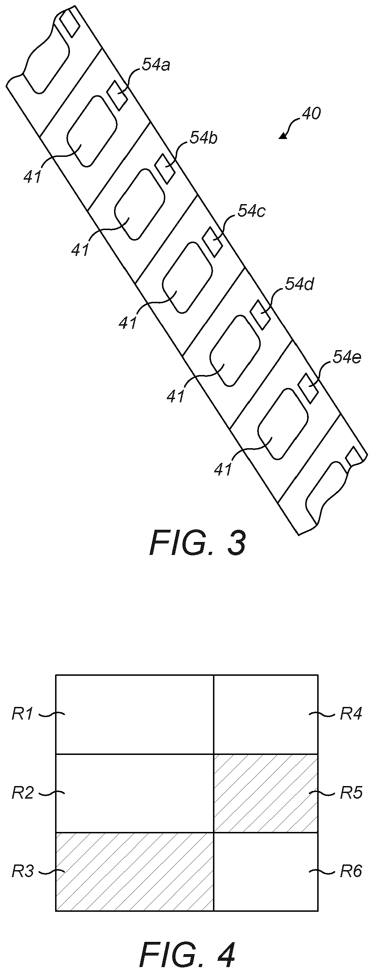

Figure 3 shows a blister strip **40** having a plurality of blisters **41** containing powdered medicament. A series of 2D matrix codes **54a, 54b, 54c, 54d, 54e,** for example a QR^{®} code, is printed onto the blister strip. The matrix codes provide indicia that define an individual number associated with each blister **41.**

Figure 4 shows an indicium in the form of a 2D matrix code which defines an individual number for a blister in a 60 dose blister strip. The code consists of three rows and two columns which define six regions denoted **R1** - **R6.** The regions are either printed black to form rectangular blocks, or left blank so that the reflective metal foil is exposed. The regions encode the digits of a 6 digit binary number. This is sufficient to define individual numbers for each blister in a 60 dose blister strip since 2⁶ = 64. In the first column, **R1** and **R2** are blank while **R3** is printed; in the second column, **R4** and **R6** are blank, and **R5** is printed.

The three optical sensors in the monitor each read one row of the code, and there are two read events as the blister strip is indexed, one for each column. However other configurations are equally possible, such as a code with two rows and three columns and a monitor with two sensors and three read events, or a 2 x 2 code for a blister strip with 15 blisters.

As shown in Figure 1E, the light guides **23** protrude into the aperture **11** in the housing of the inhaler, so that the sensors **22** are held in a fixed position relative to the blister strip. As the user actuates the inhaler, the blister strip is indexed so that the next indicium **Z** that is to be read moves past the sensors. Instantaneous readings from the sensors are taken at two specific angles during opening of the cover. The code is printed in the appropriate location on the blister strip so that each column is adjacent to the aperture in the housing when its read event occurs, i.e. at the two specific opening angles.

The sensor outputs that result from the code of Figure 4 are shown in Table 1. The monitor checks whether the output voltage from the sensor is above or below a threshold, and assigns a 0 or 1 accordingly. In Table 1, black results in a voltage below the threshold, which is designated as 1, so the indicium encodes the binary number 001010, i.e. ten. However, it would equally be possible to designate black as 0, in which case the printing of the blocks would be reversed.

**Table 1**

| **Region** | R1 | R2 | R3 | R4 | R5 | R6 |
|---|---|---|---|---|---|---|
| **Blocks** | | | | | | |
| **Voltage c.f. threshold** | Above | Above | Below | Above | Below | Above |
| **Binary number** | 0 | 0 | 1 | 0 | 1 | 0 |

In Figure 4, the indicium is printed using black ink, and the monitor detects the difference between the non-reflective ink and the reflective strip. However, the indicia could alternatively be printed using grey (as well as black) to provide a reflectance intermediate between those of the unprinted and black printed regions. Two threshold voltages could be used to distinguish three states (0, 1 and 2). This has the advantage that each block contains more information, so fewer blocks are required in each indicium. On the other hand using only black printing provides greater robustness to variations in the reflected intensities.

Alternatively, a different type of coating may be used, for example, one that is fluorescent, together with a suitable optical sensor in the monitor. Another possibility is to form the indicia by creating bumps and / or dimples in the blister strip, or to mark the strip by laser ablation. These result in different amounts of reflected light compared to unmarked parts of the blister strip.

The blister strip may additionally have printed numbers. Each individual blister is associated with one of the numbers and one of the matrix codes. However, neither the number nor the bar code is located adjacent to the blister with which it is associated. The reason for this is apparent from Figure 1E, which shows the inhaler when the first dose is about to be taken.

The blister strip has a leading end without any blisters, and then three empty blisters **44** before the first blister **X** filled with medicament. This is necessary because the indexing mechanism advances the blister strip by means of a drive wheel **7** which is located downstream of the piercer **8.** The first filled blister **X** is situated directly beneath the mouthpiece **5** and the piercer **8.** The number **Y** associated with this blister is 60, because this is the total number of doses in the inhaler before use. However, the opening **6** through which the user reads the number of doses remaining is not located adjacent to the mouthpiece, but in the sidewall of the housing close to the drive wheel **7.** Consequently, the number **Y** associated with blister **X** is actually four blisters further along the blister strip in the downstream direction. Similarly, the sensors **22** which read the indicium **Z** associated with blister **X** are located at the opposite sidewall of the housing by the aperture 11. Thus the indicium **Z** is actually four blisters upstream of blister **X** and eight blisters upstream of the number **Y.** Indicia associated with the empty blisters may be used for control checks during production.

As shown in Figure 1E, the chamber inside the inhaler that contains the blister strip is separated into two compartments by a slidably mounted dividing wall **9.** The first compartment **10A** (to the right of the dividing wall in Figure 1E) contains the unused portion of the blister strip **40** in the form of a coil. The second compartment **10B** will contain the used portion of the blister strip. As more of the blisters are used, the coil of unused blisters in the first compartment **10A** becomes smaller. At the same time, the used blister strip enters the second blister compartment **10B** and forms a coil which presses against the dividing wall as it expands. The dividing wall slides towards the unused coil, thereby enlarging the second compartment **10B** for the used blisters. The aperture **11** through which the blister strip code is read is located in the housing on the side of the first compartment **10A.** The curvature of the part of the blister strip which is read by the optical sensor varies as the unused coil decreases in size. This results in small variations in the orientation of the blister strip with respect to the optical sensor, and in the distance between the blister strip and the optical sensor, over the lifetime of the inhaler. Consequently, the amount of light reflected from the blister strip to the sensor can be different for successive indicia, which could lead to errors in reading the code.

The present invention addresses the problem of reading the code when the distance and the angle between the blister strip and the sensor can vary over the lifetime of the inhaler by optimizing the shape of the light guide.

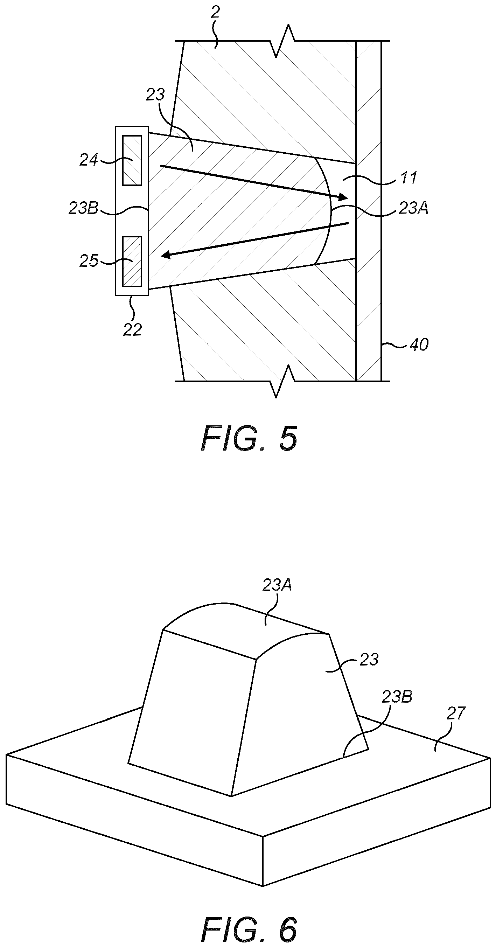

Figure 5 shows an optical sensor **22** and a light guide **23** according to the invention. The sensor **22** has an infra-red (IR) light emitting diode (LED) **24** and an IR detector **25,** such as a photodiode. The LED and detector operate in the same wavelength range, for example 940nm. Alternatively, an emitter and detector that operate in a different part of the electromagnetic spectrum, such as visible light, can be used. The sensor may be a photomicrosensor which emits and detects light. Alternatively, there may be a single LED and two or more detectors; using a single LED reduces the power consumption.

The light guide **23** channels light from the LED **24** to the blister strip **40** and back to the detector **25** as indicated by the arrows. The light travels approximately perpendicularly to the blister strip **40,** thereby reducing reflections from the housing of the inhaler and the housing of the monitor. The light guide is made from a material that is transparent to IR / visible radiation, for example a transparent plastic such as polycarbonate or acrylic (also known as polymethylmethacrylate or plexiglass).

The end of the light guide that is adjacent to the optical sensor **23B** (the proximal end) is wider than the end that is inserted into the aperture **23A** (the distal end) in the direction parallel to the blister strip (i.e. the vertical direction in Figure 5). This has the advantage that the aperture **11** can be smaller than the footprint of the sensor **22,** thereby minimizing the likelihood of objects being inserted through the aperture if the monitor is not present.

Figure 6 shows a perspective view of the light guide **23** which has a generally trapezoidal shape with a convex curved end **23A.** In theory, it would be expected that a light guide with a planar end would provide the optimum efficiency, since all of the light that reaches the end of the light guide would be incident on the blister strip (apart from Fresnel reflection losses). Moreover, the light reflected from the blister strip is diffuse (i.e. it is incident onto the end of the light guide from many directions), so it cannot be focussed by a lens. Consequently, a curved (e.g. lensed) end would not be expected to increase the amount of reflected light that re-enters the light guide. However, the present inventors have actually found that a light guide with a convex curved end results in an improved signal to noise ratio. Without wishing to be limited by theory, it is believed that the curved end compensates for variations in the angle of the blister strip (i.e. when the blister strip is not exactly perpendicular to the main axis of the light guide). Thus the curved end allows greater tolerance of variations in the orientation of the blister strip relative to the optical sensor that occur as the coil of the unused blister strip changes in size over the lifetime of the inhaler.

It would also be expected that a cuboidal light guide (i.e. with parallel sides) would provide the most efficient transfer of light from the LED to the blister strip and back again, because this shape has the greatest internal reflection. However, the present inventors have actually found that an improved signal-to-noise ratio is obtained with a light guide that has a trapezoidal shape. Without wishing to be limited by theory, it is believed that the tapering sides allow greater tolerance of variations in the position of the indicia on the blister strip relative to the position of the optical sensor that occur as the coil of the unused blister strip changes in size over the lifetime of the inhaler.

The radius of curvature of the distal end of the light guide is suitably from 2 to 5mm, such as about 3mm. The light guide suitably has a width (i.e. the size in the direction parallel to the blister strip) of from 2 to 3 mm, such as about 2.4 mm at the proximal end and from 1 to 1.5 mm, such as about 1.2 mm at the distal end. The light guide may have a depth (i.e. the size perpendicular to the blister strip) of from 1 to 3 mm, such as about 1.7 mm. The depth may be constant, or the depth (like the width) may taper from the proximal end to the distal end. The light guide may have a height (i.e. the distance from the proximal end to the distal end) of from 2 to 3 mm, such as about 2.5 mm.

The monitor shown in Figure 2B has three light guides, one for each of the optical sensors. The three light guides may be moulded as a single piece together with a base 27 as shown in Figure 6. This simplifies assembly of the monitor. Alternatively, the light guides could be formed as separate pieces and mounted in a separate base, which need not be transparent, but this would be more complex to assemble.

### Example

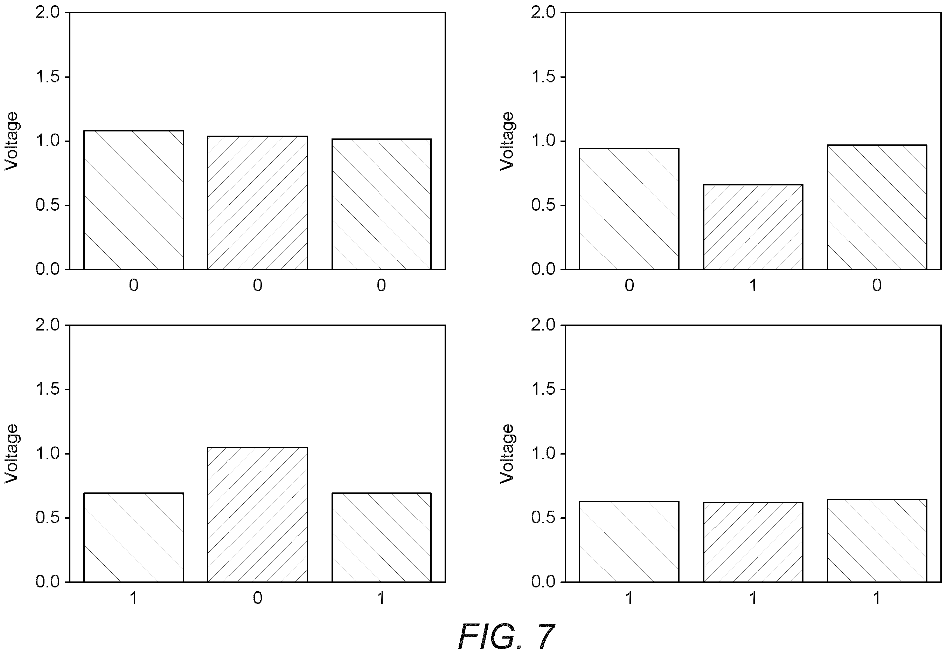

Experiments were conducted to compare three different light guide designs: a rectangular cross-section, a trapezoidal cross-section with a planar end and a trapezoidal cross-section with a convex curved end. Three optical sensors and three corresponding light guides were arranged to read a 3 x 1 matrix code on a blister strip. The light guides were inserted into the aperture of an inhaler of the type shown in Figure 1. Each light guide was used to read four different indicia on the blister strip. The indicia encoded the binary numbers 000, 010, 101 and 111). The output voltages from the optical sensors were recorded for each indicium, using each of the light guides in turn. Figure 7 shows the measured voltages for each of the four indicia recorded using the light guide with a trapezoidal cross-section and a flat end.

The differences between the measured voltages for 0 and 1 were calculated for the codes which contain both 0 and 1, i.e. 010 and 101. The light guides were then ranked in order of the voltage differences, from smallest (i.e. worst signal to noise ratio) to largest (i.e. best signal to noise ratio). The order was: the rectangular cross-section, the trapezoidal cross-section with a flat end, and the trapezoidal cross-section with a curved end. Thus the trapezoidal light guide with the curved end gave the best signal to noise ratio.

## Claims

1. A monitor (20) which is attachable to a dry powder inhaler (1) that contains a blister strip (40) having a plurality of blisters (41) and a plurality of non-numerical indicia (54), the monitor comprising at least one optical sensor (22) which is configured to read the non-numerical indicia, wherein the optical sensor has a light guide (23) having a proximal end (23B) adjacent to the sensor and a distal end (23A) remote from the sensor, wherein the distal end is convex.

2. A monitor according to claim 1 wherein the light guide has a generally trapezoidal cross-section, apart from the convex distal end.

3. A monitor according to claim 2 wherein the ratio of the width of the proximal end to the width of the distal end is from 4:3 to 3:1

4. A monitor according to claim 3 wherein the width of the proximal end is from 2 to 3 mm and the width of the distal end is from 1 to 1.5 mm.

5. A monitor according to any of claims 1 to 4 wherein the convex distal end has a radius of curvature of from 2 to 5 mm, preferably about 3mm.

6. A monitor according to any of claims 1 to 5 wherein the light guide has a height of from 2 to 3 mm.

7. A monitor according to any of claims 1 to 6 which is removably attachable to the inhaler.

8. A monitor according to any of claims 1 to 7 wherein the optical sensor comprises one or more photomicrosensors which emit and detect light.

9. A monitor according to any of claims 1 to 7 wherein the optical sensor comprises a single light source and two or more photodetectors.

10. A monitor according to any of claims 1 to 9 wherein the optical sensor operates in the visible and / or infrared regions of the electromagnetic spectrum.

11. A monitor according to any of claims 1 to 10 wherein the light guide is made from a transparent plastic material.

12. A monitor according to any of claims 1 to 11 comprising three photomicrosensors and three light guides, preferably wherein the light guides are moulded as a single component.

13. A kit comprising a monitor according to any of claims 1 to 12 and a dry powder inhaler which contains a blister strip comprising a plurality of blisters, wherein the blister strip has non-numerical indicia, and wherein the inhaler has an aperture (11) into which the light guide(s) (23) are inserted when the monitor is mounted onto the inhaler.

14. A combination comprising a monitor according to any of claims 1 to 12 mounted on a dry powder inhaler which contains a blister strip comprising a plurality of blisters, wherein the blister strip has non-numerical indicia, and wherein the light guide(s) (23) on the monitor are inserted into an aperture (11) on the inhaler, so that the optical sensors can read the non-numerical indicia.

15. A kit according to claim 13 or a combination according to claim 14 wherein the non-numerical indicia each encode an individual, unique number that is associated with a dose of powdered medicament.

## Patentansprüche

1. Monitor (20), der an einem Trockenpulverinhalator (1) anbringbar ist, der einen Blisterstreifen (40) mit einer Vielzahl von Blistern (41) und einer Vielzahl von nicht-numerischen Zeichen (54) enthält, wobei der Monitor mindestens einen optischen Sensor (22) umfasst, der ausgelegt ist, um die nicht-numerischen Zeichen zu lesen, wobei der optische Sensor einen Lichtleiter (23) mit einem proximalen Ende (23B) benachbart zu dem Sensor und einem distalen Ende (23A) aufweist, wobei das distale Ende konvex ist.

2. Monitor nach Anspruch 1, wobei der Lichtleiter einen allgemein trapezförmigen Querschnitt aufweist, abgesehen von dem konvexen distalen Ende.

3. Monitor nach Anspruch 2, wobei das Verhältnis der Breite des proximalen Endes zu der Breite des distalen Endes 4:3 bis 3:1 beträgt.

4. Monitor nach Anspruch 3, wobei die Breite des proximalen Endes 2 bis 3 mm beträgt und die Breite des distalen Endes 1 bis 1,5 mm beträgt.

5. Monitor nach einem der Ansprüche 1 bis 4, wobei das konvexe distale Ende einen Krümmungsradius von 2 bis 5 mm, vorzugsweise etwa 3 mm aufweist.

6. Monitor nach einem der Ansprüche 1 bis 5, wobei der Lichtleiter eine Höhe von 2 bis 3 mm aufweist.

7. Monitor nach einem der Ansprüche 1 bis 6, der abnehmbar an dem Inhalator anbringbar ist.

8. Monitor nach einem der Ansprüche 1 bis 7, wobei der optische Sensor einen oder mehrere Photomikrosensoren umfasst, die Licht emittieren und detektieren.

9. Monitor nach einem der Ansprüche 1 bis 7, wobei der optische Sensor eine einzige Lichtquelle und zwei oder mehr Photodetektoren umfasst.

10. Monitor nach einem der Ansprüche 1 bis 9, wobei der optische Sensor in den sichtbaren und / oder infraroten Bereichen des elektromagnetischen Spektrums arbeitet.

11. Monitor nach einem der Ansprüche 1 bis 10, wobei der Lichtleiter aus einem transparenten Kunststoffmaterial hergestellt ist.

12. Monitor nach einem der Ansprüche 1 bis 11, der drei Photomikrosensoren und drei Lichtleiter umfasst, wobei die Lichtleiter vorzugsweise als eine einzige Komponente geformt sind.

13. Kit, umfassend einen Monitor nach einem der Ansprüche 1 bis 12 und einen Trockenpulverinhalator, der einen Blisterstreifen enthält, der eine Vielzahl von Blistern umfasst, wobei der Blisterstreifen nicht-numerische Zeichen aufweist, und wobei der Inhalator eine Öffnung (11) aufweist, in die der oder die Lichtleiter (23) eingesetzt wird/werden, wenn der Monitor auf dem Inhalator montiert wird.

14. Kombination, umfassend einen Monitor nach einem der Ansprüche 1 bis 12, der auf einem Trockenpulverinhalator montiert ist, der einen Blisterstreifen enthält, der eine Vielzahl von Blistern umfasst, wobei der Blisterstreifen nicht-numerische Zeichen aufweist, und wobei der oder die Lichtleiter (23) auf dem Monitor in eine Öffnung (11) auf dem Inhalator eingesetzt ist/sind, so dass die optischen Sensoren die nicht-numerischen Zeichen lesen können.

15. Kit nach Anspruch 13 oder eine Kombination nach Anspruch 14, wobei die nicht-numerischen Zeichen jeweils eine individuelle, eindeutige Zahl codieren, die mit einer Dosis eines pulverförmigen Medikaments assoziiert ist.

## Revendications

1. Moniteur (20) qui peut être fixé à un inhalateur de poudre sèche (1) qui contient une bande d'emballages-coques (40) comportant une pluralité d'emballages-coques (41) et une pluralité de caractères non numériques (54), le moniteur comprenant au moins un capteur optique (22) qui est conçu pour lire les caractères non numériques, dans lequel le capteur optique comporte un conduit de lumière (23) ayant une extrémité proximale (23B) adjacente au capteur et une extrémité distale (23A) distante du capteur, dans lequel l'extrémité distale est convexe.

2. Moniteur selon la revendication 1 dans lequel le conduit de lumière a une section transversale généralement trapézoïdale, à l'exception de l'extrémité distale convexe.

3. Moniteur selon la revendication 2 dans lequel le rapport entre la largeur de l'extrémité proximale et la largeur de l'extrémité distale est de 4:3 à 3:1.

4. Moniteur selon la revendication 3 dans lequel la largeur de l'extrémité proximale est de 2 à 3 mm et la largeur de l'extrémité distale est de 1 à 1,5 mm.

5. Moniteur selon l'une quelconque des revendications 1 à 4 dans lequel l'extrémité distale convexe a un rayon de courbure de 2 à 5 mm, de préférence d'environ 3 mm.

6. Moniteur selon l'une quelconque des revendications 1 à 5 dans lequel le conduit de lumière a une hauteur de 2 à 3 mm.

7. Moniteur selon l'une quelconque des revendications 1 à 6 qui peut être fixé de façon amovible à un inhalateur.

8. Moniteur selon l'une quelconque des revendications 1 à 7 dans lequel le capteur optique comprend un ou plusieurs microcapteurs optiques qui émettent et détectent de la lumière.

9. Moniteur selon l'une quelconque des revendications 1 à 7 dans lequel le capteur optique comprend une seule source de lumière et au moins deux photodétecteurs.

10. Moniteur selon l'une quelconque des revendications 1 à 9 dans lequel le capteur optique fonctionne dans les régions visible et/ou infrarouge du spectre électromagnétique.

11. Moniteur selon l'une quelconque des revendications 1 à 10 dans lequel le conduit de lumière est fabriqué à partir d'un matériau plastique transparent.

12. Moniteur selon l'une quelconque des revendications 1 à 11 comprenant trois microcapteurs optiques et trois conduits de lumière, de préférence dans lequel les conduits de lumière sont moulés sous la forme d'un seul composant.

13. Kit comprenant un moniteur selon l'une quelconque des revendications 1 à 12 et un inhalateur de poudre sèche qui contient une bande d'emballages-coques comprenant une pluralité d'emballages-coques, dans lequel la bande d'emballages-coques comporte des caractères non numériques, et dans lequel l'inhalateur comporte une ouverture (11) à l'intérieur de laquelle le(s) conduit(s) de lumière (23) est/sont inséré(s) quand le moniteur est monté sur l'inhalateur.

14. Combinaison comprenant un moniteur selon l'une quelconque des revendications 1 à 12 monté sur un inhalateur de poudre sèche qui contient une bande d'emballages-coques comprenant une pluralité d'emballages-coques, dans laquelle la bande d'emballages-coques comporte des caractères non numériques, et dans laquelle le(s) conduit(s) de lumière (23) sur le monteur est/sont inséré(s) à l'intérieur d'une ouverture (11) sur l'inhalateur, de sorte que les capteurs optiques peuvent lire les caractères non numériques.

15. Kit selon la revendication 13 ou combinaison selon la revendication 14 dans lesquels les caractères non numériques encodent chacun un numéro individuel unique qui est associé à une dose de médicament en poudre.
